# EUROPÄISCHE PATENTSCHRIFT

(11) **EP 1 189 694 B1**
(45) Veröffentlichungstag und Bekanntmachung des Hinweises auf die Patenterteilung: **22.06.2011**
(21) Anmeldenummer: 00910867.1
(22) Anmeldetag: 21.03.2000
(51) Int. Cl.: B01J 23/58, C07C 67/055

(54) **KATALYSATOREN FÜR DIE GASPHASENOXIDATION VON ETHYLEN UND ESSIGSÄURE ZU VINYLACETAT, VERFAHREN ZU IHRER HERSTELLUNG UND IHRE VERWENDUNG**
CATALYSTS FOR THE GAS-PHASE OXIDATION OF ETHYLENE AND ACETIC ACID TO VINYL ACETATE AND METHOD FOR THE PRODUCTION AND USE THEREOF
CATALYSEURS PERMETTANT L'OXYDATION EN PHASE GAZEUSE DE L'ETHYLENE ET DE L'ACIDE ACETIQUE EN ACETATE DE VINYLE, LEUR FABRICATION ET LEUR UTILISATION

(30) Priorität: 27.03.1999 DE 19914066
(43) Veröffentlichungstag der Anmeldung: 27.03.2002
(73) Patentinhaber: Celanese Chemicals Europe GmbH, 60439 Frankfurt am Main (DE)
(72) Erfinder: HAGEMEYER, Alfred, Sunnyvale, CA 94086 (US); WERNER, Harald, D-61350 Bad Homburg (DE); DINGERDISSEN, Uwe, D-64342 Seeheim-Jugenheim (DE); KÜHLEIN, Klaus, D-65779 Kelkheim (DE); DAMBECK, Günter, D-65618 Selters (DE); GEISS, Gerhardt, D-65835 Liederbach (DE); RUTSCH, Andrea, D-64546 Mörfelden (DE); WEIDLICH, Stephan, D-65934 Frankfurt (DE)
(74) Vertreter: Kador & Partner
(86) Internationale Anmeldenummer: PCT/EP2000/002455
(87) Internationale Veröffentlichungsnummer: WO 2000/058008

(56) Entgegenhaltungen:
- EP-A- 0 565 952
- EP-A- 0 672 453
- EP-A- 0 723 810
- EP-A- 0 839 793

## Beschreibung

Die vorliegende Erfindung betrifft hochselektive Katalysatoren für die Vinylacetatsynthese aus Ethylen und Essigsäure, Verfahren zu ihrer Herstellung sowie ihre Verwendung.

Es ist bekannt, Vinylacetat (VAM) in der Gasphase aus Ethylen, Essigsäure und Sauerstoff oder Sauerstoff enthaltenden Gasen herzustellen; die für diese Synthese verwendeten Trägerkatalysatoren enthalten Pd als Aktivmetall und ein Alkalielement als Promotor, vorzugsweise K in Form des Acetats. Als weitere Zusätze werden Cd, Au oder Ba verwendet.

Nach US-A-4 902 823, US-A- 3 939 199, US-A-4 668 819 werden die katalytisch aktiven Komponenten in feiner Verteilung durch Tränken, Aufsprühen, Aufdampfen, Tauchen oder Ausfällen auf den Katalysatorträger aufgebracht. Nach der beschriebenen Arbeitsweise werden Katalysatoren erhalten, bei denen die aktiven Komponenten bis in den Kern des Trägers vorgedrungen sind.

Bekannt sind aber auch Katalysatoren, bei denen die aktiven Komponenten nicht bis zum Kern vorgedrungen sind, sondern nur in einen mehr oder weniger großen äußeren Teil der Trägerteilchen, d.h. die sogenannte Schale der Trägerteilchen (EP-A-0 565 952, EP-A-0 634 214, EP-A-0 634 209, EP-A-0 634 208).

Die aus dem Stand der Technik bekannten Katalysatoren enthalten als Träger die bekannten inerten Trägermaterialien wie Kieselsäure, Aluminiumoxid, Alumosilikate, Silikate, Titanoxid, Siliciumcarbid und Kohle und bei ihrer Herstellung werden die imprägnierten Katalysatorvorstufen einer Reduktion, z. B. in der Gasphase bei Temperaturen von 150-170°C beispielsweise mit Ethylen oder H₂, oder in der Flüssigphase bei Temperaturen < 100°C z.B. mit Hydrazin, unterworfen. Höhere Reduktionstemperaturen werden bewußt vermieden, da bei Temperaturen > 200°C bereits merkliche Sinterung der Edelmetallteilchen zu größeren Agglomeraten mit geringerer katalytischer Aktivität einsetzt.

Es ist aber auch bekannt, daß die mit den katalytisch aktiven Komponenten imprägnierten Träger im Zuge des Herstellverfahrens für die fertigen Katalysatoren höheren Temperaturbereichen ausgesetzt werden können. So beschreiben US-A-5 336 802 und US-A-5 194 417 die Behandlung von Palladium- und Gold-haltigen Katalysatoren durch einfache und mehrfache sequentielle Oxidations- und Reduktionsschritte.

EP-A-0 839 793 betrifft ein Verfahren zur Herstellung von Vinylacetat in Gegenwart eines Palladium-haltigen Katalysators, bei dessen Herstellung der imprägnierte und mit einem Reduktionsmittel behandelte Katalysatorvorläufer einem zusätzlichen Sinterungsschritt bei einer Temperatur zwischen 500 und 1000°C unterworfen wird.

Aus WO 98/ 18553 ist bekannt, einen Palladium- und Gold-enthaltenden Katalysator nach dem Imprägnierungsschritt bei einer Temperatur von 100 bis 600°C in einer nicht-reduzierenden Atmosphäre zu calcinieren und erst anschließend den Reduktionsschritt vorzunehmen. Die Calcinierung kann dabei auch in einer Sauerstoffatmosphäre vorgenommen werden.

Auch bei den in EP-A-0 839 793 und WO 98/ 18553 offenbarten Verfahren erfolgt die Vinylacetatherstellung unter Verwendung von Trägerkatalysatoren, die auf inerten Trägermaterialien wie Kieselsäure, Aluminiumoxid, Alumosilikate, Silikate, Titanoxid, Siliciumcarbid und Kohle beruhen.

Vor dem Hintergrund, daß Katalysatoren in großtechnisch durchgeführten Prozessen, wie der Vinylacetatherstellung, Verwendung finden, besteht ein großes Interesse daran, die Katalysatoren, insbesondere hinsichtlich ihrer Aktivität und Selektivität zu verbessern.

Es war daher eine Aufgabe der vorliegenden Erfindung, Katalysatoren für die Vinylacetatsynthese zur Verfügung zustellen, die sich durch eine besonders hohe Aktivität, Selektivität und Standzeit auszeichnen.

Des weiteren war es Aufgabe der vorliegenden Erfindung Katalysatoren bereitzustellen, die sich durch eine besonders hohe Stabilität auszeichnen. Insbesondere sollten die Katalysatoren gegenüber lokalen Temperaturschwankungen und punktueller Überhitzung ("hot spots") sowie gegen Schwankungen der Sauerstoffkonzentration während der Reaktionsführung in der Vinylacetat-Synthese und gegenüber mechanischer Beanspruchung unempfindlich sein.

Gelöst werden diese Aufgaben durch einen Katalysator, der im Anspruch 1 beschrieben ist.

Der Gegenstad der Erfindundung betrifft einem katalysator wie im Anspruch 1 beschrieben ein Verfahren beschrieben im Anspruch 7 zur dessen Herstellung sowie dessen Verwendung wie im Anspruch 13 beschrieben. Weitere Merkmale der Erfindung sind in den Worlaut der abhängigen Ansprüche 2-6 und 8-12 enthalten.

Im folgenden wird die Reduktion des mit Palladiumverbindungen beladenen, porösen Trägers, der mindestens ein reduzierbares Metalloxid enthält, bei einer Temperatur von >200°C als Hochtemperaturreduktion bezeichnet und mit HTR abgekürzt.

Wesentlich für die erfindungsgemäßen Katalysatoren ist die Verwendung eines Trägermaterials, das mindestens ein reduzierbares Metalloxid enthält, das aus Titandioxid (TiO₂) besteht.

Trägermaterialen, wie Kieselsäure, Aluminiumoxid, Aluminiumsilikate, Siliciumcarbid oder Kohle sind aus EP-A- 723 810 bekannt. Der Gehalt an dem reduzierbaren Metalloxide TiO₂ beträgt dabei 0,1 bis 25 Gew.-%, bezogen auf das gesamte Trägermaterial.

Ebenfalls kann man Mischungen der reduzierbaren Metalloxide mit diesen inerten Materialien als Träger für die erfindungsgemäßen Katalysatoren verwenden. Dabei kann der Anteil an den inerten Materialien bis zu 75 Gew.-%, bezogen auf das gesamte Trägermaterial, betragen. Vorzugsweise beträgt der Anteil an den inerten Materialien bis zu 50 Gew.-%, bezogen auf das gesamte Trägermaterial.

Trägermaterialien, die mindestens das reduzierbare Metalloxid TiO₂ enthalten, werden im folgenden als reduzierbares Trägermaterial bezeichnet.

Überraschenderweise sind aus dem Stand der Technik bekannte, nicht reduzierbare Trägermaterialien, wie beispielsweise Kieselsäure oder Aluminiumoxid oder deren Mischungen zur Herstellung der erfindungsgemäßen Katalysatoren ungeeignet. Ebenso ungeeignet sind Oxide als Trägermaterialien, die sich unter den erfindungsgemäß angewandten Herstellungsbedingungen zum Metall reduzieren lassen.

Der reduzierbare Träger kann sowohl in Pulverform als auch als Formkörper eingesetzt werden. Bevorzugt wird das Trägermaterial als Formkörper eingesetzt und liegt in Form von Pellets, Kugeln, Tabletten, Ringen, Strängen, Rippsträngen, Sternsträngen, Sternen, Hohlextrudaten oder anderen technischen Formkörpern vor. Der Durchmesser bzw. die Länge und Dicke der Trägerteilchen liegt im allgemeinen bei 3 bis 9 mm. Die Oberfläche der Träger liegt, gemessen mit der BET-Methode bei 15 - 250 m²/g. Das Porenvolumen liegt bei 0.2 bis 1.2 ml/g. Die Bestimmung des Porenvolumens erfolgt mittels der Quecksilberporosimetrie.

Die erfindungsgemäßen Trägermaterialien können auf jede dem Fachmann bekannte Weise hergestellt werden. Sie sind im allgemeinen kommerziell erhältlich.

Dadurch, daß man einen reduzierbaren Träger mit mindestens einer Palladiumverbindung belädt und anschließend eine Hochtemperaturreduktion bei Temperaturen von 300-600°C durchführt und vor oder nach der Reduktion mindestens eine Alkalimetallverbindung und gegebenenfalls einen oder mehrere Promotoren zusätzlich aufgibt, werden Katalysatoren erhalten, die sich durch eine hervorragende Aktivität und Selektivität sowie eine ausgezeichnete Edelmetallhaftung auf dem Träger auszeichnen.

Die erfinderische Maßnahme der HTR mit Palladiumverbindungen beladenen porösen reduzierbaren Trägern wird bei Temperaturen 300-600°C durchgeführt.

Auf den so beladenen Träger gibt man gegebenenfalls anschließend einen oder mehrere Promotoren zusätzlich auf. Vorzugsweise verwendet man mindestens eine Au- , Ba- , oder Cd-Verbindung oder Mischungen dieser Verbindungen.

Vorzugsweise erfolgt der Promotorenzusatz jedoch vor der HTR-Behandlung, und zwar entweder im Gemisch mit der oder den Palladiumverbindungen oder getrennt davon, wobei die Reihenfolge keine Rolle spielt, ob der poröse reduzierbare Träger zunächst mit der Palladiumverbindung und dann mit den Promotoren beladen wird oder umgekehrt.

Zusätzlich enthalten die erfindungsgemäßen Katalysatoren mindestens eine Alkalimetallverbindung, vorzugsweise mindestens eine Kaliumverbindung. Der Alkalimetallzusatz zu dem porösen reduzierbaren Träger kann vor der HTR-Behandlung erfolgen, entweder separat oder im Gemisch mit den übrigen Promotoren und/oder mit der oder den Palladiumverbindungen oder nach der HTR-Behandlung, entweder separat oder im Gemisch mit den übrigen Promotoren.

Schließlich können die erfindungsgemäßen Katalysatoren entsprechend dem Stand der Technik konfektioniert werden und in großtechnischen Prozessen, wie zum Beispiel in der Vinylacetatsynthese, verwendet werden.

Erfindungsgemäß wird das reduzierbare Trägermaterial zunächst mit einer Palladiumverbindungen beladen. Die Art der Verbindung ist hierbei unkritisch, solange eine hohe Dispersion des Metalls erzielt werden kann. Hierbei sind lösliche Palladiumverbindungen geeignet, insbesondere wasserlösliche Salze. Bevorzugt sind beispielsweise Palladiumverbindungen aus einer Gruppe, welche Palladium(II)-acetat, Palladium(II)-chlorid, Tetrachloropalladium(II)säure-Natriumsalz [Na₂PdCl₄] und Palladium(II)-nitrat umfaßt. Neben Palladium-(II)-acetat lassen sich weitere Carboxylate des Palladiums verwenden, vorzugsweise die Salze der aliphatischen Monocarbonsäuren mit 3 bis 5 Kohlenstoffatomen, beispielsweise das Propionat oder Butyrat.

Im Falle der Chloride muß jedoch sichergestellt werden, daß die Chloridionen vor dem Einsatz des Katalysators auf eine tolerable Restmenge reduziert werden. Dazu wird der Katalysatorträger nach der Beladung mit Palladium-verbindungen und mit den Promotoren und nach der HTR mit Wasser gewaschen. Dies geschieht durch Auswaschen des Trägers, z.B. mit Wasser, nachdem Pd und ggf. Au durch Reduktion zu den Metallen auf dem Träger fixiert wurden.

Die genannten Palladiumverbindungen können auf jede dem Fachmann bekannte Weise hergestellt werden. Sie sind im allgemeinen aber auch kommerziell erhältlich.

Als Promotoren verwendet man Verbindungen der Elemente Au, Ba und Cd, insbesondere lösliche Salze dieser Verbindungen.

Zu den geeigneten Goldverbindungen gehören beispielsweise Tetrachloro-gold(III)säure [HAuCl₄], Gold(III)acetat [Au(OAc)₃], Kaliumaurat [KAuO₂]. Es ist empfehlenswert, das Au-Acetat bzw. das K-Aurat durch Fällung des Oxids/Hydroxids aus Goldsäure-Lösungen, Waschung und Isolierung des Niederschlages und Aufnahme in Essigsäure, beziehungsweise KOH jeweils frisch anzusetzen. Die Goldverbindungen werden bevorzugt vor der Hochtemperaturreduktion auf den Träger aufgebracht.

Zu den geeigneten Cadmiumverbindungen gehören beispielsweise Cadmiumacetat Cd(OAc)₂ und andere Carboxylate des Cadmiums, wie zum Beispiel das Propionat oder Butyrat. Die Cadmiumverbindungen können sowohl vor als auch nach der Hochtemperaturreduktion auf den Träger aufgebracht werden.

Zu den geeigneten Bariumverbindungen gehören beispielsweise Bariumacetat Ba(OAc)₂, andere Bariumcarboxylate, wie zum Beispiel das Propionat oder Butyrat sowie Bariumhydroxid Ba(OH)₂. Die Bariumverbindungen können sowohl vor als auch nach der Hochtemperaturreduktion auf den Träger aufgebracht werden.

Zusätzlich enthält der erfindungsgemäße Katalysator mindestens eine Alkalimetallverbindung, vorzugsweise mindestens eine Kalium- oder Rubidiumverbindung und besonders bevorzugt mindestens eine Kaliumverbindung. Zu den geeigneten Kaliumverbindungen gehören beispielsweise Kaliumacetat KOAc, Kaliumcarbonat K₂CO₃, Kaliumhydrogencarbonat KHCO₃ und Kaliumhydroxid KOH, sowie sämtliche Kaliumverbindungen, die sich unter Reaktionsbedingungen in das Acetat umwandeln. Die Kaliumverbindungen können sowohl vor als auch nach der Hochtemperaturreduktion auf den Träger aufgebracht werden.

Bevorzugt werden für die Beladung des porösen reduzierbaren Trägers die Acetate verwendet, da diese die Katalysatoren nur in sehr geringem Umfang mit Chlorid kontaminieren.

Die Metallverbindungen werden üblicherweise in Konzentrationen von etwa 0,1 bis 100 g pro Liter, vorzugsweise 1 bis 50 g pro Liter, bezogen auf das Lösungsmittel, eingesetzt.

Als Lösungsmittel sind alle Verbindungen geeignet, in denen die gewählten Verbindungen bzw. Salze löslich sind und die nach der Imprägnierung leicht wieder durch Trocknung zu entfernen sind. Beispielsweise sind für die Acetate vor allem unsubstituierte Carbonsäuren, insbesondere Essigsäure, geeignet, wohingegen die Chloride vor allem in Wasser oder verdünnter Salzsäure löslich sind.

Falls die Salze in der Essigsäure oder im Wasser nicht genügend löslich sind, kann neben Wasser bzw. Essigsäure, die auch als Mischung eingesetzt werden, die zusätzliche Verwendung eines weiteren Lösungsmittels zweckmäßig sein.

Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, Acetonitril, Dimethylformamid, aber auch Kohlenwasserstoffe wie Benzol.

Die Belegung mit der Aktivkomponente Pd und den weiteren Promotoren sowie mit der Alkalimetallverbindung kann nach den bekannten Methoden des Standes der Technik erfolgen.

Die fertigen Katalysatoren weisen folgende Metallgehalte, ausgedrückt in Gramm Metall, bezogen auf 1 I fertigen Katalysator, auf:

| | | |
|---|---|---|
| Palladium: | im allgemeinen | 1 - 20 g/l |
| | vorzugsweise | 3 - 15 g/l |
| | insbesondere | 5 - 10 g/l |
| | | |
| Alkalimetallgehalt: | im allgemeinen | 5 - 30 g/l |
| | vorzugsweise | 10 - 25 g/l |
| | insbesondere | 10 - 16 g/l |

Falls der fertige Katalysator zusätzlich einen oder mehrere Promotoren enthält, so liegen die Gehalte des Promotors jeweils im allgemeinen bei bis zu 20 g/l, vorzugsweise bei 2 - 15 g/l und insbesondere bei 3 - 10 g/l.

Vorzugsweise werden in der Vinylacetatsynthese die Katalysatorsysteme auf Basis Pd/Cd/K, Pd/Ba/K oder Pd/Au/K eingesetzt. Besonders bevorzugt sind Systeme, die Pd-Au-K enthalten. Zwingend für die vorliegende Erfindung ist, daß wenigstens Pd und ggf. Au der HTR ausgesetzt werden, während die anderen Promotoren sowie die Kaliumverbindungen und weitere Zusätze sowohl vor als auch nach der HTR zugegeben werden können.

Es zeigt sich, daß die erfindungsgemäßen Katalysatoren den aus dem Stand der Technik bekannten Katalysatoren hinsichtlich Aktivität und besonders hinsichtlich der Selektivität in großtechnischen Prozessen überlegen sind. Ein solcher, wichtiger großtechnischer Prozeß ist zum Beispiel die Vinylacetatsynthese.

Der Einsatz der erfindungsgemäßen Katalysatoren in der Vinylacetatsynthese zeichnet sich insbesondere durch folgende Vorteile aus:

Überraschenderweise wird eine Selektivitätserhöhung um mehr als 5% erreicht, die durch eine drastische Verminderung der unerwünschten Totaloxidation zu CO₂ meßbar ist.

Gleichzeitig wird eine Aktivitätserhöhung um mehr als 20% im Vergleich zu den aus dem Stand der Technik bekannten Katalysatoren erzielt.

Durch die erfindungsgemäße Maßnahme der HTR wird eine sehr starke Wechselwirkung der Edelmetallteilchen mit dem reduzierbaren Träger induziert, die die Eigenschaften der Edelmetallteilchen für die Katalyse vorteilhaft verändert und für eine ausgezeichnete mechanische Verankerung auf dem Träger und damit eine hohe Agglomerationsresistenz verantwortlich ist.

Die erfindungsgemäßen hochtemperaturreduzierten Katalysatoren weisen eine besonders gleichmäßige Pd/Au-Aktivmetall-Verteilung und hohe Edelmetall-Dispersion auf.

Die hohe Dispersion bleibt selbst im Dauerbetrieb aufgrund verminderter Agglomeration der Edelmetallteilchen weitgehend erhalten, wodurch die Deaktivierung der erfindungsgemäßen Katalysatoren verlangsamt wird und lange Standzeiten erzielt werden.

Ferner weisen die erfindungsgemäßen Katalysatoren ausgezeichnete mechanische Stabilitäten auf, da die chemisch reaktiven reduzierbaren Träger leicht zu mechanisch harten Formkörpern verformt werden können und die HTR bei hohen Temperaturen die Härte der Formlinge obendrein noch erhöht.

Die erfindungsgemäßen Katalysatoren sind unempfindlich gegen punktuelle Überhitzungen ("hot spots") sowie Schwankungen der Sauerstoffkonzentration während der Reaktionsführung der Vinylacetat-Synthese, wobei die hot-spots aufgrund der außerordentlich hohen Selektivität (drastische Verminderung der stark exothermen Totaloxidation zu CO oder CO₂) stark vermindert auftreten, wodurch die Regelung und Prozeßkontrolle deutlich vereinfacht wird.

Da eine Steigerung der Raum-Zeit-Ausbeute (RZA) mit einer gleichzeitigen Selektivitätserhöhung einhergeht, können besonders hohe Durchsätze in Großanlagen technisch realisiert werden.

Das Erzielen der obengenannten Vorteile war aus dem Stand der Technik nicht absehbar, da in dem Stand der Technik die Meinung vertreten wird, daß Reduktionstemperaturen > 200°C schädlich sind und aufgrund von Agglomerationsphänomenen der Edelmetallteilchen die Aktivität senken. Daher werden sowohl bei der herkömmlichen Reduktion als auch später im Betrieb hot-spots > 190°C tunlichst vermieden.

Ferner lehrt der Stand der Technik die Verwendung von inerten Trägern, wie zum Beispiel Siliciumdioxid oder Siliciumdioxid/Aluminiumoxid-Gemische, vorzugsweise Siliciumdioxid.

Die Beladung des Trägers mit den gewünschten Mengen an den jeweiligen Verbindungen kann in einem oder mehreren sequentiellen Schritten erfolgen, wobei zwischen den einzelnen Schritten gegebenenfalls Trocknungsschritte eingefügt werden können.

Von jedem der auf die Trägerteilchen aufzubringenden Elemente, wie z.B. Pd/K/Au, Pd/K/Cd oder Pd/K/Ba, kann beispielsweise eine Verbindung, wie z.B. ein Salz, aufgebracht werden. Es können aber auch mehrere Salze eines Elements aufgebracht werden oder Mischverbindungen der verschiedenen Metalle eingesetzt werden. Im allgemeinen verwendet man von jedem der drei Elemente genau ein Salz.

Die Salze können gemäß bekannter Beladungsverfahren, wie beispielsweise Tränken, Imprägnieren, Aufsprühen, Aufdampfen, Tauchen oder Ausfällen auf den Träger aufgebracht werden. Der Träger kann mit den Salzen durchimprägniert werden oder es können alle dem Fachmann bekannte Verfahren angewandt werden, um Schalenkatalysatoren zu erzeugen. Diese werden z.B. in den Dokumenten DE-A-1 668 088, US-PS 3 775 342, US-PS 3 822 308, US-PS 4 048 096, US-PS 5 185 308, US-PS 5 567 839, US-PS 5 314 858, EP-A-0 634 208, EP-A-0 634 209 oder EP-A-0 634 214 dargelegt.

Bei den Pd/Au/K-Katalysatoren hat es sich als vorteilhaft erwiesen, die beiden Edelmetalle in Form einer Schale auf den Träger aufzubringen, d.h. die Edelmetalle sind nur in einer oberflächennahen Zone verteilt, während die weiter innen liegenden Bereiche des Trägerformkörpers nahezu edelmetallfrei sind. Die Schichtdicke dieser katalytisch aktiven Schalen beträgt gewöhnlich 0.1 - 2 mm.

Mit Hilfe von Schalenkatalysatoren ist in vielen Fällen eine selektivere Verfahrensdurchführung möglich als mit Katalysatoren, bei denen die Trägerteilchen bis in den Kern imprägniert ("durchimprägniert") sind.

Läßt man die Reaktionsbedingungen bei Verwendung von Schalenkatalysatoren gegenüber den Reaktionsbedingungen bei Verwendung von durchimprägnierten Katalysatoren unverändert, so läßt sich mehr Vinylacetat pro Reaktorvolumen und Zeit herstellen, was einer Kapazitätserweiterung ohne zusätzlichen Investitionsaufwand gleichkommt. Es wird auch die Aufarbeitung des erhaltenen Rohvinylacetats erleichtert, da der Vinylacetatgehalt im Produktgas des Reaktors höher ist, was weiterhin zu einer Energieersparnis während der Aufarbeitung führt. Geeignete Aufarbeitungsverfahren werden z.B. in US-PS 5 066 365, DE-34 22 575, DE-A-34 08 239, DE-A-29 45 913, DE-A-26 10 624, US-PS 3 840 590 beschrieben.

Hält man dagegen die Anlagenkapazität konstant, so kann man die Reaktionstemperatur senken und dadurch bei gleicher Gesamtleistung die Reaktion selektiver durchführen, wobei Edukte eingespart werden. Dabei wird auch die Menge des als Nebenprodukt entstehenden und daher auszuschleusenden Kohlendioxids und der mit dieser Ausschleusung verbundene Verlust an mitgeschlepptem Ethylen geringer. Darüberhinaus führt diese Fahrweise zu einer Verlängerung der Katalysatorstandzeit.

Erfindungsgemäß muß der Katalysator nach der Beladung mit mindestens einer Palladiumverbindung einer Hochtemperaturreduktion unterzogen werden.

Hierfür kann beispielsweise ein verdampfbares oder gasförmiges Reduktionsmittel bei Temperaturen im Bereich von 300°C bis 600°C über den beladenen Katalysator geleitet werden.

Als Reduktionsmittel für die HTR sind alle Stoffe geeignet, die die Palladium-und gegebenfalls Goldverbindungen bei den verwendeten erfindungsgemäßen hohen Reduktionstemperaturen zu den Metallen zu reduzieren vermögen.

Gasförmige oder verdampfbare Reduktionsmittel, wie beispielsweise H₂ ,CO, Ethylen, NH₃, Formaldehyd, Methanol und Kohlenwasserstoffe im allgemeinen sowie Mischungen dieser Reduktionsmittel, sind bevorzugt. Wasserstoff ist hierbei besonders bevorzugt.

Die gasförmigen Reduktionsmittel können auch mit Inertgas, wie beispielsweise Kohlendioxid, Stickstoff oder Argon verdünnt sein. Vorzugsweise wird ein mit Inertgas verdünntes Reduktionsmittel eingesetzt. Bevorzugt sind Mischungen von Wasserstoff mit Stickstoff oder Argon, vorzugsweise mit einem Wasserstoffgehalt zwischen 1 Vol% und 15 Vol%.

Die Reduktionszeiten liegen im allgemeinen im Bereich von 1 Minute bis 24 Stunden, besonders bevorzugt 30 Minuten bis 10 Stunden.

Die Menge des Reduktionsmittels wird so gewählt, daß während der Behandlungsdauer mindestens das zur vollständigen Reduktion der Edelmetalle nötige Äquivalent Reduktionsmittel über den Katalysator geleitet wird. Bevorzugt wird ein Überschuß an Reduktionsmittel über den Katalysator geleitet, um eine vollständige Reduktion zu gewährleisten. Aus dem bei der Reduktion angewendeten Druck, der Verdünnung und der Reaktionszeit ergibt sich der Volumenstrom an Reduktiongas. Vorzugsweise wird drucklos, d.h. bei einem Absolutdruck von ca. 1 bar reduziert. Für die Herstellung technischer Mengen an erfindungsgemäßem Katalysator wird bevorzugt ein Drehrohrofen oder ein Wirbelschichtreaktor verwendet, um eine gleichmäßige Reduktion des Katalysators zu gewährleisten.

Nach der erfindungsgemäßen Methode werden in erster Linie die Edelmetallverbindungen, also Pd und beispielsweise Au, zu den entsprechenden Metallen reduziert, wobei das Trägermaterial anreduziert wird, z.B. unter Bildung von Ti³⁺-Zentren im TiO₂-Gitter. Im Gegensatz dazu werden die übrigen anwesenden Metallverbindungen, d.h. die Alkalimetallverbindungen und die übrigen Promotoren, außer Gold, im allgemeinen nicht reduziert. Daher können die Alkalimetallverbindungen und die nicht reduzierbaren Promotoren sowohl vor als auch nach der Hochtemperaturreduktion auf den Träger aufgebracht werden.

In einer bevorzugten Ausführungsform der Erfindung werden TiO₂-Träger oder deren Mischungen oder Abmischungen mit weiteren inerten Trägerbestandteilen, wie zum Beispiel SiO₂ und/oder Al₂O₃ vorzugsweise als Formkörper, bevorzugt in Form von Pellets, Kugeln, Ringen, Strängen, Tabletten, mit Pd-Acetat und Au-Acetat imprägniert, getrocknet, der HTR im Temperaturbereich von 300 - 600°C für einen Zeitraum im Bereich von 1 Minute bis 10 Stunden in der Gasphase mit gasförmigen Reduktionsmitteln, vorzugsweise H₂, Ethylen und/oder NH₃, umgesetzt. Gegebenenfalls schließt sich dann eine Imprägnierung mit K-Acetat sowie eine abschließende Trocknung bei einer Temperatur von höchstens 150°C, bevorzugt 80-150°C und insbesondere 100-150°C an.

Man kann aber auch solche Trägermaterialien verwenden, bei denen die inerten Trägerbestandteile zunächst mit TiO₂ beschichtet wurden. Anschließend erfolgt der Imprägnierschritt und die HTR. Der TiO₂-Anteil beträgt dabei 0,1 bis 25 Gew.-%, bezogen auf das Trägermaterial.

Weitere Vor- oder Nachbehandlungsschritte, die dem Fachmann bekannt sind, können eingefügt werden. Hierzu gehören unter anderem Waschen, Trocknen, Calcinieren, Oxidieren und/oder Reduzieren.

Bevorzugt werden die erfindungsgemäßen Katalysatoren zur Herstellung von Vinylacetat verwendet. Diese erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drücken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird. Das entstandene Vinylacetat wird mit Hilfe geeigneter Methoden isoliert, die beispielsweise in US-PS 5 066 365, DE-A-34 22 575, DE-A-34 08 239, DE-A-29 45 913, DE-A-26 10 624, US-PS 3 840 590 beschrieben werden.

Die nachfolgenden Beispiele dienen zur eingehenderen Erläuterung und Veranschaulichung der Erfindung, ohne daß diese darauf eingeschränkt sein soll.

### Beispiel 1

2,11 g Palladiumacetat (224,49 g/Mol) und 1,32 g Goldacetat (374,10 g/Mol) wurden in 30 ml Eisessig gelöst. Die Herstellung des Goldacetats ist beispielsweise in US 4.933.204 beschrieben. Zu dieser Lösung wurden 100 ml TiO₂-Träger (P25 Pellets, Firma DEGUSSA) gegeben. Anschließend wurde zunächst ein Großteil des Eisessigs am Rotationsverdampfer bei 70°C abdestilliert, danach wurden Reste des Lösungsmittels bei 60°C mit Hilfe einer Ölpumpe und schließlich in einem Vakuumtrockenschrank ebenfalls bei 60°C über einen Zeitraum von 14 Stunden entfernt.

Die Reduktion erfolgte mit einer Gasmischung von 10 Vol% H₂ in N₂. Hierbei wurde das Gas bei einer Temperatur von ca. 500°C für 1 Stunde durch die Pellets geleitet. Die Reduktion wurde unter Normaldruck bei einem Fluß von 40 l/h eines Gemisches von 10 Vol% H₂ in Stickstoff durchgeführt. Zur Beladung mit Kaliumionen wurden die Pellets mit einer Lösung von 4 g Kaliumacetat in 30 ml Wasser versetzt. Diese Mischung ließ man bei Raumtemperatur für 15 Minuten in einem Mischer auf die Pellets einwirken. Anschließend wurde das Lösungsmittel am Rotationsverdampfer entfernt. Die Pellets wurden über 14 Stunden bei 110°C im Trockenschrank getrocknet.

Der Katalysator enthielt: 7 g/l Au; 16 g/l K; <1 g/l Cl; 10 g/l Pd.

Die Herstellung des Katalysators wurde zweimal wiederholt. Die Austestung dieser Katalysatoren in der Vinylacetatsynthese ist in der nachfolgenden Tabelle 1 mit 1A bis 1C wiedergegeben.

### Beispiel 2

2,11 g Palladiumacetat (224,49 g/Mol) und 1,32 g Goldacetat (374,10 g/Mol) wurden in 45 ml Eisessig gelöst. Zu dieser Lösung wurden 100 ml TiO₂-Träger (XT25376 Pellets, Firma Norton) gegeben. Anschließend wurde zunächst ein Großteil des Eisessigs am Rotationsverdampfer bei 70°C abdestilliert, danach wurden Reste des Lösungsmittels bei 60°C mit Hilfe einer Ölpumpe und schließlich in einem Vakuumtrockenschrank ebenfalls bei 60°C über einen Zeitraum von 14 Stunden entfernt.

Die Reduktion erfolgte mit einer Gasmischung bestehend aus 10 Vol% H₂ in N₂. Hierbei wurde das Gas bei einer Temperatur von ca. 500°C für 1 Stunde durch die Pellets geleitet. Die Reduktion wurde unter Normaldruck bei einem Fluß von 40 l/h eines Gemisches von 10 Vol% H₂ in Stickstoff durchgeführt. Zur Beladung mit Kaliumionen wurden die Pellets mit einer Lösung von 4 g Kaliumacetat in 45 ml Wasser versetzt. Die so getränkten Pellets wurden bei Raumtemperatur für 10 Minuten durchmischt. Anschließend wurde das Lösungsmittel am Rotationsverdampfer entfernt. Die Pellets wurden über 14 Stunden bei 110°C im Trockenschrank getrocknet.

Der Katalysator enthielt: 7 g/l Au; 16 g/l K; <1,1 g/l Cl; 10 g/l Pd.

### Beispiele 3A-C

2,11 g Palladiumacetat (224,49 g/Mol), 1,32 g Goldacetat (374,10 g/Mol) und 4,0g Kaliumacetat wurden in 30 ml Eisessig gelöst. Zu dieser Lösung wurden 100 ml TiO₂-Träger (P25 Pellets, Firma DEGUSSA) gegeben. Anschließend wurde zunächst ein Großteil des Eisessigs am Rotationsverdampfer bei 60°C abdestilliert, danach wurden Reste des Lösungsmittels bei 60°C in einem Vakuumtrockenschrank in 4 Stunden entfernt.

Der Ansatz wurde in drei Teile geteilt, die unterschiedlichen Reduktionsbedingungen unterzogen wurden.

| Ansatz | Zeit | Temperatur | Reduktionsmittel |
|---|---|---|---|
| A | 4 h | 400°C | 10 Vol% H₂ in N₂ |
| B | 4 h | 450°C | 10 Vol% H₂ in N₂ |
| C | 4 h | 500°C | 10 Vol% H₂ in N₂ |

Die Reduktion erfolgte mit einer Gasmischung bestehend aus 10 Vol% H₂ in N₂. Hierbei wurde das Gas bei einer Temperatur von ca. 400°C (Ansatz A), 450°C (Ansatz B) bzw. 500°C (Ansatz C), jeweils für 4 Stunden durch die Pellets geleitet. Die Reduktion wurde unter Normaldruck bei einem Fluß von 40 l/h durchgeführt.

Der Katalysator enthielt: 7 g/l Au; 16 g/l K; <1,5 g/l Cl; 10 g/l Pd.

### Beispiel 4

1,06g g Palladiumacetat (224,49 g/Mol), 0,66 g Goldacetat (374,10 g/Mol) und 2,0 g Kaliumacetat wurden in 15 ml Eisessig gelöst. Zu dieser Lösung wurden 50 ml TiO₂-Träger (P25 Pellets, Firma DEGUSSA) gegeben. Anschließend wurde zunächst ein Großteil des Eisessigs am Rotationsverdampfer bei 60°C abdestilliert, danach wurden Reste des Lösungsmittels über 14 Stunden in einem Vakuumtrockenschrank bei 60°C entfernt.

Die Reduktion erfolgte mit einer Gasmischung bestehend aus 10 Vol% H₂ in N₂ bei 500°C für 1 Stunde analog zu Beispiel 1.

Der Katalysator enthielt: 7 g/l Au; 16 g/l K; <1 g/l Cl; 10 g/l Pd.

### Beispiel 5

1,06 g Palladiumacetat (224,49 g/Mol), 0,66 g Goldacetat (374,10 g/Mol) und 4,0 g Kaliumacetat wurden in 30 ml Eisessig bei 60°C gelöst. Zu dieser Lösung wurden 100 ml TiO₂-Träger (P25 Pellets, Firma DEGUSSA) gegeben. Anschließend wurde zunächst ein Großteil des Eisessigs am Rotationsverdampfer bei 60°C abdestilliert, danach wurden Reste des Lösungsmittels mit einer Ölpumpe über einen Zeitraum von 4h bei 60°C entfernt.

Die Reduktion erfolgte mit einer Gasmischung bestehend aus 10 Vol% H₂ in N₂ bei 500°C für 1 Stunde analog zu Beispiel 1.

Der Katalysator enthielt: 3,5 g/l Au; 16 g/l K; <0,5 g/l Cl; 5 g/l Pd.

### Vergleichsbeispiel 1

1,82 g Na₂PdCl₄ (294,19 g/Mol) und 0,64 g NaAuCl₄ (361,76 g/Mol) wurden in 32 ml demineralisiertem Wasser gelöst. Diese Lösung wurde unter leichter Bewegung auf 100 ml SiO₂-Träger (KA160 Pellets, Firma Süd-Chemie) vollständig aufgetragen. Zur Ausbildung einer Edelmetallschale wurde der vorbehandelte Träger in eine Lösung von 0,85 g Natriumhydroxid, NaOH, in 32 ml demineralisiertes Wasser gegeben. Die Reaktionsmischung wurde über Nacht ruhengelassen und danach mit demineralisiertem Wasser chloridfrei gewaschen.

Danach wurde der Katalysator 5 Stunden bei 150°C mit einem Ethylen/Stickstoff-Gemisch (5% Ethylen in Stickstoff) reduziert.

Zur Beladung mit Kaliumionen wurden die Pellets mit einer Lösung von 4 g Kaliumacetat in 30 ml Wasser versetzt und der fertige Katalysator 2 Stunden im Schnelltrockner getrocknet.

Der Katalysator enthielt: 3,5 g/l Au; 16 g/l K; <0,5 g/l Cl; 6,6 g/l Pd.

### Vergleichsbeispiel 2

1,06 g Palladiumacetat (224,49 g/Mol), 0,7 g Goldacetat (374,10 g/Mol) und 4,0 g Kaliumacetat wurden in 30 ml Eisessig bei 60°C gelöst. Zu dieser Lösung wurden 100 ml TiO₂-Träger (P25 Pellets, Firma DEGUSSA) gegeben. Anschließend wurde zunächst ein Großteil des Eisessigs am Rotationsverdampfer bei 60°C abdestilliert, danach wurden Reste des Lösungsmittels mit einer Ölpumpe über einen Zeitraum von 4h bei 60°C entfernt.

Die Reduktion erfolgte mit einer Gasmischung bestehend aus 10 Vol.% H₂ und 90 Vol.% N₂, jeweils bezogen auf das Volumen, bei 170°C für 1 Stunde(Normaldruck, Gasfluß: 40 l/h).

Der Katalysator enthielt: 3,5 g/l Au; 16 g/l K; <0,5 g/l Cl; 5 g/l Pd.

### Vergleichsbeispiel 3

0,53 g Palladiumacetat (224,49 g/Mol), 0,33 g Goldacetat (374,10 g/Mol) und 2,0 g Kaliumacetat wurden in 30 ml Eisessig gelöst. Zu dieser Lösung wurden 50 ml TiO₂-Träger (XT25376 Pellets, Firma Norton) gegeben. Anschließend wurde zunächst ein Großteil des Eisessigs am Rotationsverdampfer bei 70°C abdestilliert, danach wurden Reste des Lösungsmittels bei 60°C mit Hilfe einer Ölpumpe und schließlich in einem Vakuumtrockenschrank ebenfalls bei 60°C über einen Zeitraum von 14 Stunden entfernt.

Die Reduktion erfolgte thermisch, ohne Reduktionsgas (Autoreduktion). Hierbei wurde Stickstoff als Spülgas bei einer Temperatur von ca. 500°C für 1 Stunde durch die Pellets geleitet (Normaldruck, 40 l/h).

Der Katalysator enthielt: 3,5 g/l Au; 16 g/l K; <0,5 g/l Cl; 5 g/l Pd.

### Vergleichsbeispiel 4

1,06 g Palladiumacetat (224,49 g/Mol), 0,7 g Goldacetat (374,10 g/Mol) und 4,0 g Kaliumacetat wurden in 80 ml Eisessig bei 60°C gelöst. Zu dieser Lösung wurden 100 ml SiO₂-Träger (Aerosil 200 Pellets, Firma DEGUSSA) gegeben. Anschließend wurde zunächst ein Großteil des Eisessigs am Rotationsverdampfer bei 60°C abdestilliert, danach wurden Reste des Lösungsmittels mit einer Ölpumpe über einen Zeitraum von 4h bei 60°C entfernt.

Die Reduktion erfolgte mit einer Gasmischung bestehend aus 10 Vol% H₂ in N₂ bei 500°C für 1 Stunde analog zu Beispiel 1.

Der Katalysator enthielt: 3,5 g/l Au; 16 g/l K; <0,5 g/l Cl; 5 g/l Pd.

### Vergleichsbeispiel 5

1,06 g Palladiumacetat (224,49 g/Mol), 0,7 g Goldacetat (374,10 g/Mol) und 4,0 g Kaliumacetat wurden in 80 ml Eisessig bei 60°C gelöst. Zu dieser Lösung wurden 100 ml SiO₂-Träger (KA160 Pellets, Firma Süd-Chemie) gegeben. Anschließend wurde zunächst ein Großteil des Eisessigs am Rotationsverdampfer bei 60°C abdestilliert, danach wurden Reste des Lösungsmittels mit einer Ölpumpe über einen Zeitraum von 4h bei 60°C entfernt.

Die Reduktion erfolgte mit einer Gasmischung bestehend aus 10 Vol% H₂ in N₂ bei 500°C für 1 Stunde analog zu Beispiel 1.

Der Katalysator enthielt: 3,5 g/l Au; 16 g/l K; <0,5 g/l Cl; 5 g/l Pd.

### Reaktortests für die Gasphasenoxidation von Ethylen und Essigsäure zu Vinylacetat:

Die in den Beispielen und Vergleichsbeispielen hergestellten Katalysatoren werden in einem Festbett-Rohrreaktor mit 2 cm Rohrduchmesser getestet. Der Reaktor wird von außen mit einer Öl-Mantelheizung temperiert. Es werden typischerweise 15 ml der Katalysator-Formkörper vorgelegt. Das Reaktorvolumen vor und nach der Katalysatorschüttung wird zur Verringerung des Totvolumens mit Glaskugeln aufgefüllt. Die Gasdosierung erfolgt über Massenflußregler für Gase, die Essigsäure wird mit einer Massenflußregler-Verdampfereinheit dosiert. Die Mischung der Gase und der Essigsäure erfolgt in einem mit Füllkörpern beschickten Gasmischrohr. Die Testapparatur wird kontinuierlich betrieben.

Die Reaktion wird ständig mit dem Gaschromatographen überwacht.

Bei gleichmäßiger Reaktion, d. h. bei konstanter Reaktortemperatur und gleichbleibender Konzentration von Vinylacetat und CO₂ im Produktgasstrom, beginnt die Datenaufzeichnung.

Bei den Versuchen wurde eine Reaktionstemperatur im Bereich von 150-170°C sowie ein Reaktionsdruck von 8-9 bar verwendet. Der Eduktstrom setzte sich typischerweise aus 60 - 80 Vol.-% Ethylen, 10 - 20 Vol.-% N₂ , 10-20 Vol.-% Essigsäure und 2 - 10 Vol.-% O₂ zusammen. Eine Vollanalyse des Reaktoraustrags wurde direkt am Reaktorausgang mittels on-line GC (2 Säulen-Schaltung) sowie on-line IR durchgeführt.

Aus den GC-Daten wurden die Vinylacetat-Selektivitäten S (= Mol VAM/(Mol VAM + 0.5 * Mol CO_{x,} X= 1 oder 2) und RZA (Raum-Zeit-Ausbeute = g VAM / l Kat. * h) bestimmt. Die Ergebnisse der einzelnen Versuche sind in Tabelle 1 aufgeführt, wobei die in den Beispielen 1 bis 5 hergestellten Katalysatoren getestet wurden.

**Tabelle 1: Austestung der Katalysatoren in der Vinylacetat-Synthese**

| Beispiel Nr. | T (°C) | p (bar) | O₂-Konz (%) | S % | RZA g/l*h |
|---|---|---|---|---|---|
| 1A | 170 | 9 | 5,2 | 96 | 1000 |
| | 160 | 9 | 5,2 | 98 | 1050 |
| | 155 | 9 | 5,2 | 98 | 1000 |
| 1B | 170 | 9 | 5,2 | 97 | 700 |
| | 160 | 9 | 5,2 | 98 | 1150 |
| 1C | 170 | 9 | 5,2 | 98 | 1300 |
| 2 | 170 | 9 | 5,2 | 96 | 1200 |
| 3A | 160 | 9 | 5,2 | 89 | 1400 |
| | 150 | 9 | 5,2 | 98 | 1400 |
| 3B | 160 | 9 | 5,2 | 95 | 1260 |
| 3C | 160 | 9 | 5,2 | 96 | 1210 |
| 4 | 150 | 9 | 5,2 | 96 | 1100 |
| 5 | 160 | 9 | 5,2 | 95 | 940 |
| Vgl. 1 | 170 | 9 | 5,1 | 88 | 850 |
| Vgl. 2 | 160 | 9 | 5,2 | 80 | 870 |
| Vgl. 3 | 170 | 9 | 5,2 | 77 | < 50 |
| Vgl. 4 | 167 | 9 | 5,2 | 89 | 190 |
| Vgl. 5 | 170 | 9 | 5,2 | 83 | 340 |

Die Tabelle belegt, daß durch die HTR-Behandlung von Katalysatoren, die ein reduzierbares Trägermaterial enthalten, die Selektivität und Ausbeute in der Vinylacetat-Synthese deutlich verbessert werden im Vergleich zu den aus dem Stand der Technik bekannten Katalysatoren.

## Patentansprüche

1. Katalysator, der Palladium, mindestens eine Alkalimetallverbindung und gegebenenfalls einen oder mehrere Promotoren auf einem porösen Träger enthält, **dadurch** erhältlich, daß man den porösen Träger, der aus TiO₂ besteht und der eine Oberfläche von 15-250 m²/g und ein Porenvolumen von 0,2 bis 1,2 ml/g aufweist, mit mindestens einer Palladiumverbindung belädt, anschließend eine Reduktion bei einer Temperatur von 300-600°C durchführt und wobei man vor oder nach der Reduktion mindestens eine Alkalimetallverbindung und gegebenenfalls einen oder mehrere Promotoren zusätzlich aufgibt.

2. Katalysator gemäß Anspruch 1, **dadurch gekennzeichnet, daß** der Katalysator mindestens eine Kaliumverbindung enthält.

3. Katalysator gemäß Anspruch 1 oder 2, **dadurch gekennzeichnet, daß** der Katalysator zusätzlich als Promotoren Au, Ba und/oder Cd und/oder deren Verbindungen enthält.

4. Katalysator gemäß einem oder mehreren der Ansprüche 1 bis 3, **dadurch gekennzeichnet, daß** die Reduktion für eine Zeitdauer im Bereich von 1 Minute bis 24 Stunden erfolgt.

5. Katalysator gemäß einem oder mehreren der Ansprüche 1 bis 4, **dadurch gekennzeichnet, daß** die Reduktion mit gasförmigen oder verdampfbaren Reduktionsmitteln erfolgt.

6. Katalysator gemäß einem oder mehreren der Ansprüche 1 bis 5, **dadurch gekennzeichnet, daß** das Reduktionsmittel für die Reduktion ausgewählt ist aus der Gruppe umfassend H₂, CO, Ethylen, NH₃, Formaldehyd, Methanol Kohlenwasserstoffe sowie deren Mischungen oder Mischungen dieser Reduktionsmittel mit inerten Gasen.

7. Verfahren zur Herstellung von Katalysatoren, **dadurch gekennzeichnet, daß** man den porösen Träger, der aus TiO₂ besteht und der eine Oberfläche von 15-250 m²/g und ein Porenvolumen von 0,2 bis 1,2 ml/g aufweist, mit mindestens einer Palladiumverbindung belädt, anschließend eine Reduktion bei einer Temperatur von 300-600°C durchführt und wobei man vor oder nach der Reduktion mindestens eine Alkalimetallverbindung und gegebenenfalls einen oder mehrere Promotoren zusätzlich aufgibt.

8. Verfahren nach Anspruch 7, **dadurch gekennzeichnet, daß** der Katalysator mindestens eine Kaliumverbindung enthält.

9. Verfahren gemäß Anspruch 7 oder 8, **dadurch gekennzeichnet, daß** der Katalysator zusätzlich als Promotoren Au, Ba und/oder Cd und/oder deren Verbindungen enthält.

10. Verfahren gemäß einem oder mehreren der Ansprüche 7 bis 9, **dadurch gekennzeichnet, daß** die Reduktion für eine Zeitdauer im Bereich von 1 Minute bis 24 Stunden erfolgt.

11. Verfahren gemäß einem oder mehreren der Ansprüche 7 bis 10, **dadurch gekennzeichnet, daß** die Reduktion mit gasförmigen oder verdampfbaren Reduktionsmitteln erfolgt.

12. Verfahren gemäß einem oder mehreren der Ansprüche 7 bis 11, **dadurch gekennzeichnet, daß** das Reduktionsmittel für die Reduktion ausgewählt ist aus der Gruppe umfassend H₂, CO, Ethylen, NH₃, Formaldehyd, Methanol Kohlenwasserstoffe sowie deren Mischungen oder Mischungen dieser Reduktionsmittel mit inerten Gasen.

13. Verwendung des nach den Ansprüchen 7-12 erhältlichen Katalysators zur Herstellung von Vinylacetat in der Gasphase aus Ethylen, Essigsäure und Sauerstoff oder Sauerstoff enthaltenden Gasen.

## Claims

1. A catalyst containing palladium, at least one alkali metal compound and optionally one or more promoters on a porous support, obtainable by loading the porous support, which consists of TiO₂ and which has a surface of 15-250 m²/g and a pore volume of 0.2 to 1.2 ml/g, with at least one palladium compound, subsequently performing a reduction at a temperature of 300-600°C, and whereby at least one alkali metal compound and optionally one or more promoters are additionally fed before or after the reduction.

2. The catalyst according to claim 1, **characterized in that** the catalyst contains at least one potassium compound.

3. The catalyst according to claim 1 or 2, **characterized in that** the catalyst additionally contains as promoters Au, Ba and/or Cd and/or compounds thereof.

4. The catalyst according to one or more of claims 1 to 3, **characterized in that** the reduction is effected for a time duration in the range of 1 minute to 24 hours.

5. The catalyst according to one or more of claims 1 to 4, **characterized in that** the reduction is effected with gaseous or vaporizable reducing agents.

6. The catalyst according to one or more of claims 1 to 5, **characterized in that** the reducing agent for the reduction is selected from the group comprising H₂, CO, ethylene, NH₃, formaldehyde, methanol, hydrocarbons and mixtures thereof or mixtures of said reducing agents with inert gases.

7. A process for preparing catalysts, **characterized in that** the porous support, which consists of TiO₂ and which has a surface of 15-250 m²/g and a pore volume of 0.2 to 1.2 ml/g, is loaded with at least one palladium compound, subsequently a reduction is performed at a temperature of 300-600°C, and whereby at least one alkali metal compound and optionally one or more promoters are additionally fed before or after the reduction.

8. The process according to claim 7, **characterized in that** the catalyst contains at least one potassium compound.

9. The process according to claim 7 or 8, **characterized in that** the catalyst additionally contains as promoters Au, Ba and/or Cd and/or compounds thereof.

10. The process according to one or more of claims 7 to 9, **characterized in that** the reduction is effected for a time duration in the range of 1 minute to 24 hours.

11. The process according to one or more of claims 7 to 10, **characterized in that** the reduction is effected with gaseous or vaporizable reducing agents.

12. The process according to one or more of claims 7 to 11, **characterized in that** the reducing agent for the reduction is selected from the group comprising H₂, CO, ethylene, NH₃, formaldehyde, methanol, hydrocarbons and mixtures thereof or mixtures of said reducing agents with inert gases.

13. Use of the catalyst obtainable according to claims 7-12 for preparing vinyl acetate in the gaseous phase from ethylene, acetic acid and oxygen or oxygen-containing gases.

## Revendications

1. Catalyseur qui contient du palladium, au moins un composé de métal alcalin et, le cas échéant, un ou plusieurs promoteurs sur un support poreux, que l'on peut obtenir en chargeant le support poreux, qui est constitué de TiO₂ et qui présente une surface de 15 à 250 m²/g et un volume de pores de 0,2 à 1,2 ml/g, avec au moins un composé de palladium, en réalisant ensuite une réduction à une température de 300 à 600 °C et en y rajoutant, avant ou après la réduction, au moins un composé de métal alcalin et, le cas échéant, un ou plusieurs promoteurs en plus.

2. Catalyseur selon la revendication 1, **caractérisé en ce que** le catalyseur contient au moins un composé de potassium.

3. Catalyseur selon la revendication 1 ou 2, **caractérisé en ce que** le catalyseur contient en outre, comme promoteurs, les éléments Au, Ba et/ou Cd et/ou leurs composés.

4. Catalyseur selon l'une quelconque ou plusieurs des revendications 1 à 3, **caractérisé en ce que** la réduction est réalisée pour une durée dans une plage de temps de 1 minute à 24 heures.

5. Catalyseur selon l'une quelconque ou plusieurs des revendications 1 à 4, **caractérisé en ce que** la réduction est effectuée avec des agents de réduction gazeux ou évaporables.

6. Catalyseur selon l'une quelconque ou plusieurs des revendications 1 à 5, **caractérisé en ce que** l'agent de réduction est choisi, pour la réduction, dans le groupe comprenant le H₂, le CO, l'éthylène, le NH₃, le formaldéhyde, le méthanol, les hydrocarbures ainsi que leurs mélanges ou des mélanges de ces agents de réduction avec des gaz inertes.

7. Procédé pour la fabrication de catalyseurs, **caractérisé en ce que** l'on charge le support poreux, qui est constitué de TiO₂ et qui présente une surface de 15 à 250 m²/g et un volume de pores de 0,2 à 1,2 ml/g, avec au moins un composé de palladium, **en ce que** l'on réalise ensuite une réduction à une température de 300 à 600 °C tout en rajoutant, avant ou après la réduction, au moins un composé de métal alcalin et, le cas échéant, un ou plusieurs promoteurs en plus.

8. Procédé selon la revendication 7, **caractérisé en ce que** le catalyseur contient au moins un composé de potassium.

9. Procédé selon la revendication 7 ou 8, **caractérisé en ce que** le catalyseur contient en outre, comme promoteurs, les éléments Au, Ba et/ou Cd et/ou leurs composés.

10. Procédé selon l'une quelconque ou plusieurs des revendications 7 à 9, **caractérisé en ce que** la réduction est réalisée pour une durée dans une plage de temps de 1 minute à 24 heures.

11. Procédé selon l'une quelconque ou plusieurs des revendications 7 à 10, **caractérisé en ce que** la réduction est effectuée avec des agents de réduction gazeux ou évaporables.

12. Procédé selon l'une quelconque ou plusieurs des revendications 7 à 11, **caractérisé en ce que** l'agent de réduction est choisi, pour la réduction, dans le groupe comprenant le H₂, le CO, l'éthylène, le NH₃, le formaldéhyde, le méthanol, les hydrocarbures ainsi que leurs mélanges ou des mélanges de ces agents de réduction avec des gaz inertes.

13. Utilisation du catalyseur qui peut être obtenu selon les revendications 7 à 12, pour la fabrication d'acétate de vinyle dans la phase gazeuse à partir d'éthylène, d'acide acétique ou de gaz contenant de l'oxygène.
